# EUROPEAN PATENT APPLICATION

(11) **EP 4 835 096 A1**
(43) Date of publication of application: **04.11.2026**
(21) Application number: 24912922.2
(22) Date of filing: 25.12.2024
(51) Int. Cl.: C12Q 1/6851, C12N 15/11, C12Q 1/02

(54) **METHOD FOR ASSISTING DIAGNOSIS OF ADVANCED ADENOMA IN LARGE INTESTINE, AND METHOD FOR ASSISTING DIAGNOSIS OF EITHER ADVANCED ADENOMA IN LARGE INTESTINE OR COLORECTAL CANCER**

(30) Priority: 25.12.2023 JP 2023217701
(71) Applicant: Yamaguchi University, Yamaguchi 753-8511 (JP); Eiken Kagaku Kabushiki Kaisha, Tokyo 101-0062 (JP)
(72) Inventor: YAMASAKI, Takahiro, Ube-shi, Yamaguchi 755-8505 (JP); SUEHIRO, Yutaka, Ube-shi, Yamaguchi 755-8505 (JP); ISHIGURO, Akiyo, Ube-shi, Yamaguchi 755-8505 (JP); KUNIMUNE, Yuki, Ube-shi, Yamaguchi 755-8505 (JP); INOUE, Yukari, Ube-shi, Yamaguchi 755-8505 (JP)
(74) Representative: Hasegawa, Kan
(86) International application number: PCT/JP2024/045944
(87) International publication number: WO 2025/143036

(57) **Abstract**

An object of the present invention is to provide a novel method capable of diagnosing advanced colorectal adenoma, or a novel method capable of diagnosing advanced colorectal adenoma or colorectal cancer.

According to one aspect, the method includes:
(i) a step A of measuring a methylation level of CpG present in a region from +1 to +300 relative to a transcription start site of a somatostatin (SST) gene in DNA extracted from feces collected from a subject;
(ii) a step B of measuring a quantitative level of a predetermined internal control gene in DNA extracted from feces collected from the subject; and
(iii) a step C of calculating a normalized methylation level by normalizing the methylation level of CpG in the SST gene measured in the step A by the quantitative level of the predetermined internal control gene measured in the step B, in which the normalized methylation level calculated in the step C is compared with a predetermined cutoff value, thereby assisting in the diagnosis of advanced colorectal adenoma in the subject.

## Description

### Technical Field

The present invention relates to a method for assisting in the diagnosis of advanced colorectal adenoma, and a method for assisting in the diagnosis of either advanced colorectal adenoma or colorectal cancer.

### Background Art

Colorectal cancer is a disease with high mortality and incidence rates in Japan. In Japan, colorectal cancer ranked first among all cancers in terms of incidence in 2019, with 155,625 cases, and ranked second in terms of the number of cancer deaths in 2021, with 52,418 deaths. It is known that, if colorectal cancer can be detected at the stage of advanced adenoma, early-stage cancer, or a precancerous lesion, the benefits of curative surgery can be obtained in approximately 95% or more of cases. Therefore, early detection and early treatment are extremely important.

A fecal occult blood test is a well-established method for screening for colorectal tumors. While the fecal occult blood test can detect colorectal cancer with high accuracy, a drawback is its low detection rate for advanced adenomas. Specifically, whereas the test sensitivity of the fecal occult blood test for colorectal cancer ranges from 65.8% to 73.8%, its test sensitivity for advanced adenomas is merely 23.8% to 27.1% (see Non-patent literatures 1 and 2).

The "Cologuard (registered trademark)" kit is a test that is described as providing improved diagnostic performance for advanced adenoma. This kit is a colorectal tumor screening kit that combines a fecal occult blood test with a fecal DNA test, and it was approved by the U.S. Food and Drug Administration (FDA) in 2014. The DNA test of "Cologuard" consists of detecting mutations in the KRAS gene, analyzing the methylation of the BMP3 and NDRG4 genes, and quantifying the copy number of the ACTB gene. While the test sensitivity of "Cologuard" for colorectal cancer stands at 92.3%, its test sensitivity for advanced adenoma remains limited to 42.4% (see the above Non-patent literature 2). Furthermore, this test requires a fecal specimen including the entire volume of a single bowel movement (about 200 g); however, in Japan, the infrastructure necessary to conduct tests using such large-volume fecal specimens is not currently in place. Moreover, when taking into account the burdens associated with hygiene and specimen transport, it would be difficult to implement this test in its current form. Additionally, the test is associated with high costs, as it requires analyzing a large number of target genes and performing gene mutation analysis and methylation analysis as separate procedures.

Meanwhile, the present inventors have previously developed a highly sensitive DNA methylation analysis technique and have reported, utilizing this technique, that the methylated Twist homolog 1 (TWIST1) gene serves as a marker for colorectal tumors (see Patent literature 1 and Non-patent literature 3). Although a fecal DNA test targeting the methylated TWIST1 gene was combined with a fecal occult blood test, the false-positive rate in the control group (the group without colorectal tumors) was a somewhat high 19.7%, indicating that there was room for improvement (see Non-patent literature 4). Furthermore, as described in the above Patent literature 1, there are as many as six CpG sites in the transcriptional regulatory region of TWIST1, and in order to cleave each CpG site, the methylation-sensitive restriction enzymes corresponding to those CpG sites, HhaI, HpaII, and BstUI, were used. Here, in the methylation-sensitive restriction enzyme treatment, performing digestion with all restriction enzymes at once tends to result in incomplete cleavage. Therefore, it was necessary to devise a method in which the restriction enzymes are added in two steps in the restriction enzyme treatment, thereby improving the cleavage efficiency of the restriction enzymes and enhancing the accuracy of methylation detection.

In addition, the present inventors have disclosed a method for predicting the presence or absence of colorectal tumors based on the detection of CpG methylation in the TWIST1, NDRG4, BMP3, or SEPT9 gene in feces or serum (see Patent literature 2), a method for determining cancer by measuring the CpG methylation level in double-stranded DNA fragments derived from the RUNX3 gene (see Patent literature 3), and the utility of a combination of CpG-methylated SEPT9 and AFP in serum for the diagnosis of hepatocellular carcinoma (see Non-patent literatures 5 and 6). Furthermore, the present inventors have disclosed a method for predicting the presence or absence of precancerous lesions or cancer based on the concentration or amount of double-stranded DNA in a biological sample, or based on the copy number of the human telomerase reverse transcriptase (hTERT) gene in a biological sample (see Patent literature 4).

Furthermore, other research groups have reported elevated levels of somatostatin (SST) gene methylation in colorectal cancer tissues (see Non-patent literature 7). However, these studies focused only on colorectal cancer, excluding advanced colorectal adenoma from their scope of studies. Moreover, the methylation status of the SST gene in feces samples had not been examined at all. Additionally, while the relationship between the SST gene and various types of cancer has been disclosed, advanced colorectal adenoma was not included in these studies (see Non-patent literature 8).

### Citation List

### Patent Literature

Patent Literature 1: WO2010/113529
Patent Literature 2: WO2017/043497
Patent Literature 3: JP2020-014415A
Patent Literature 4: JP2019-106990A

### Non-Patent Literature

Non-Patent Literature 1: Morikawa, Tamiya et al. A comparison of the immunochemical fecal occult blood test and total colonoscopy in the asymptomatic population. Gastroenterology vol. 129,2 (2005): 422-8. doi:10.1016/j.gastro.2005.05.05.
Non-Patent Literature 2: Imperiale, Thomas F et al. Multitarget stool DNA testing for colorectal-cancer screening. The New England journal of medicine vol. 370,14 (2014): 1287-97. doi:10.1056/NEJMoa1311194.
Non-Patent Literature 3: Suehiro Y, Zhang Y, Hashimoto S, et al. Highly sensitive faecal DNA testing of TWIST1 methylation in combination with faecal immunochemical test for haemoglobin is a promising marker for detection of colorectal neoplasia. Ann Clin Biochem 2018; 55: 59-68.
Non-Patent Literature 4: Suehiro Y, et al. Fecal DNA Testing of TWIST1 Methylation Identifies Patients with Advanced Colorectal Adenoma Missed by Fecal Immunochemical Test for Hemoglobin. Clinical and translational gastroenterology vol. 11,6 (2020): e00176. doi:10.14309/ctg.0000000000000176.
Non-Patent Literature 5: Kotoh Y, Suehiro Y, Saeki I, et al. Novel Liquid Biopsy Test Based on a Sensitive Methylated SEPT9 Assay for Diagnosing Hepatocellular Carcinoma. Hepatol Commun 2020; 4: 461-470.
Non-Patent Literature 6: Ayano Yamazaki et al., Liquid Biopsy Test Based on a Sensitive DNA Methylation Assay for Diagnosing Hepatocellular Carcinoma: Which Is a Better Marker, Methylated SST or SEPT9. Yamaguchi Medical Journal, Vol. 70, No. 3, pp. 89-98, 2021.
Non-Patent Literature 7: Mehdi Manoochehri, et al. SST gene hypermethylation acts as a pan-cancer marker for pancreatic ductal adenocarcinoma and multiple other tumors: toward its use for blood-based diagnosis. Mol Oncol 2020 Jun;14(6):1252-1267. doi: 10.1002/1878-0261.12684.
Non-Patent Literature 8: Mori Y, et al. A Genome-Wide Search Identifies Epigenetic Silencing of Somatostatin, Tachykinin-1, and 5 Other Genes in Colon Cancer. GASTROENTEROLOGY 2006; 131:797-808.

### Summary of the Invention

### Technical Problem

Colorectal cancer is almost completely curable if detected and treated at an early stage or at the stage of precancerous lesions. However, to date, no test method has been established that can specifically and with high accuracy detect advanced colorectal adenoma associated with a high risk of malignant transformation. As described above, the fecal occult blood test is a commonly used test method for colorectal cancer. However, a drawback of the fecal occult blood test has been its low detection rate for advanced colorectal adenoma which carries a high risk of malignancy. Accordingly, the object of the present invention is to provide a novel method capable of diagnosing advanced colorectal adenoma, or a novel method capable of diagnosing either advanced colorectal adenoma or colorectal cancer.

### Means for Solving the Problem

As a result of diligent studies aimed at solving the aforementioned problems, the present inventors have discovered that normalizing the methylation level of the SST gene in DNA extracted from feces by the quantitative level of an internal control gene serves as a useful aid in the diagnosis of advanced colorectal adenoma. Furthermore, the present inventors have found that, by performing statistical analysis using indicators including the methylation level of the SST gene in DNA extracted from feces normalized by the quantitative level of an internal control gene, and the result of a fecal occult blood test, the present method serves as a useful aid in diagnosing advanced colorectal adenoma or colorectal cancer, as compared with the fecal occult blood test alone, thereby completing the present invention.

Specifically, the present invention is as follows.
[1] A method for assisting in the diagnosis of advanced colorectal adenoma in a subject, including:
   (i) a step A of measuring a methylation level of CpG present in a region from +1 to +300 relative to a transcription start site of a somatostatin (SST) gene in DNA extracted from feces collected from a subject;
   (ii) a step B of measuring a quantitative level of a predetermined internal control gene in DNA extracted from feces collected from the subject; and
   (iii) a step C of calculating a normalized methylation level by normalizing the methylation level of CpG in the SST gene measured in the step A by the quantitative level of the predetermined internal control gene measured in the step B, in which the normalized methylation level calculated in the step C is compared with a predetermined cutoff value, thereby assisting in the diagnosis of advanced colorectal adenoma in the subject.
[2] The method according to the above [1], in which, in the step C, the normalized methylation level is calculated by dividing the methylation level of CpG in the SST gene measured in the step A by the quantitative level of the predetermined internal control gene measured in the step B.
[3] The method according to the above [1] or [2], in which the quantitative level of the internal control gene is a copy number of the internal control gene.
[4] The method according to any of the above [1] to [3], in which, in the step A, the methylation level of CpG present in the region from +1 to +300 relative to the transcription start site of the SST gene is measured by treating DNA with at least one restriction enzyme selected from the group consisting of HhaI, HpaII, BstUI, BmgT120I, AatII, BssHII, Cfr10I, HaeII, Hin1I, SacII, and VpaK11BI, and then amplifying a region containing CpG recognized by the restriction enzyme.
[5] The method according to any of the above [1] to [4], in which, in the step A, cytosine of CpG present in the region from +1 to +300 relative to the transcription start site of the SST gene is cytosine located at position 187,670,340 or 187,670,342 on human chromosome 3 (Chr3).
[6] The method according to any of the above [1] to [5], in which the subject is a subject determined to be negative by a fecal occult blood test.
[7] A method for assisting in diagnosis of advanced colorectal adenoma or colorectal cancer, including:
   (i) a step A of measuring a methylation level of CpG present in a region from +1 to +300 relative to a transcription start site of a somatostatin (SST) gene in DNA extracted from feces collected from a subject;
   (ii) a step B of measuring a quantitative level of a predetermined internal control gene in DNA extracted from feces collected from the subject;
   (iii) a step C of calculating a normalized methylation level by normalizing the methylation level of CpG in the SST gene measured in the step A by the quantitative level of the predetermined internal control gene measured in the step B; and
   (iv) a step D of performing a fecal occult blood test and determining whether the test is positive or negative based on a predetermined cutoff value, in which multivariate analysis is performed using, as indicators, the methylation level measured in the step A and/or the normalized methylation level calculated in the step C, and a result of the fecal occult blood test determined in the step D, thereby assisting in the diagnosis of advanced colorectal adenoma or colorectal cancer.
[8] The method according to the above [7], in which, in the step C, the normalized methylation level is calculated by dividing the methylation level of CpG in the SST gene measured in the step A by the quantitative level of the predetermined internal control gene measured in the step B.
[9] The method according to the above [7] or [8], in which, in the step A, the methylation level of CpG present in the region from +1 to +300 relative to the transcription start site of the SST gene is measured by treating DNA with at least one restriction enzyme selected from the group consisting of HhaI, HpaII, BstUI, BmgT120I, AatII, BssHII, Cfr10I, HaeII, Hin1I, SacII, and VpaK11BI, and then amplifying a region containing CpG recognized by the restriction enzyme.
[10] The method according to any of the above [7] to [9], in which cytosine of CpG present in the region from +1 to +300 relative to the transcription start site of the SST gene is cytosine located at position 187,670,340 or 187,670,342 on human chromosome 3 (Chr3).
[11] A method for assisting in diagnosis of advanced colorectal adenoma or colorectal cancer in a subject, including:
   (i) a step A of measuring a methylation level of CpG present in a region from +1 to +300 relative to a transcription start site of a somatostatin (SST) gene in DNA extracted from feces collected from a subject;
   (ii) a step B of measuring a quantitative level of a predetermined internal control gene in DNA extracted from feces collected from the subject;
   (iii) a step C of calculating a normalized methylation level by normalizing the methylation level of CpG in the SST gene measured in the step A by the quantitative level of the predetermined internal control gene measured in the step B;
   (iv) a step D of performing a fecal occult blood test and determining whether the test is positive or negative based on a predetermined cutoff value;
   (v) a step E of determining whether the methylation level measured in the step A is positive or negative based on a predetermined cutoff value; and
   (vi) a step F of determining whether the normalized methylation level calculated in the step C is positive or negative based on a predetermined cutoff value, in which the subject is determined, for the purpose of diagnostic assistance, as having advanced colorectal adenoma or colorectal cancer if a result determined in the step D and at least one of a result determined in the step E and a result determined in the step F, is positive.
[12] The method according to the above [11], in which, in the step C, the normalized methylation level is calculated by dividing the methylation level of CpG in the SST gene measured in the step A by the quantitative level of the predetermined internal control gene measured in the step B.

### Advantageous Effects of Invention

According to the present invention, it becomes possible to assist in the diagnosis of advanced colorectal adenoma which is likely to be missed by a colorectal cancer screening test.

### Brief Description of Drawings

FIG. 1 is a supplementary explanatory diagram of a normalized mSST value used in Examples.
FIG. 2A is a diagram illustrating the distribution of mSST values in a control group, a non-advanced adenoma group, an advanced adenoma group, and a colorectal cancer group.
FIG. 2B is a diagram illustrating the distribution of human TERT (hTERT) values in the control group, the non-advanced adenoma group, the advanced adenoma group, and the colorectal cancer group.
FIG. 2C is a diagram illustrating the distribution of the normalized mSST values in the control group, the non-advanced adenoma group, the advanced adenoma group, and the colorectal cancer group.
FIG. 3 is a diagram illustrating a result graphically represented based on the result in Table 2, categorized into the "Control group", the "Non-advanced adenoma group", the "Advanced adenoma group", and the "Colorectal cancer group".
FIG. 4 is a diagram illustrating a result in which the distribution of predicted values based on Equation (III) is plotted for the control group, the non-advanced adenoma group, and the advanced adenoma group, followed by a multiple comparison of the predicted values among these groups using Dunn's test.
FIG. 5 is a diagram illustrating a result in which the distribution of predicted values based on Equation (IV) is plotted for the control group, the non-advanced adenoma group, the advanced adenoma group, and the colorectal cancer group, followed by a multiple comparison of the predicted values among these groups using Dunn's test.
FIG. 6 is a diagram illustrating a result of ROC analysis (for a reference group (control group + non-advanced adenoma group) vs. a disease group (advanced adenoma group + colorectal cancer group)) to evaluate the ability of each prediction equation to discriminate between the reference group and the disease group.

### Modes for Carrying out the Invention

The contents described in all patent and non-patent literatures cited in the present specification are incorporated herein by reference in their entirety.

A method for assisting in the diagnosis of advanced colorectal adenoma of the present invention includes:
(i) a step A of measuring a methylation level of CpG present in a region from +1 to +300 relative to transcription start site of the somatostatin (SST) gene in DNA extracted from feces collected from a subject;
(ii) a step B of measuring a quantitative level of a predetermined internal control gene in DNA extracted from feces collected from the subject;
(iii) a step C of calculating a normalized methylation level by normalizing the methylation level of CpG in the SST gene measured in the step A by the quantitative level of the predetermined internal control gene measured in the step B, in which the normalized methylation level is compared with a predetermined cutoff value. The method is not particularly limited as long as it assists in the diagnosis of advanced colorectal adenoma in the subject in this manner. Hereinafter, the method may also be referred to as the "method for assisting in the diagnosis of advanced colorectal adenoma of the present invention".

Further, a method A for assisting in the diagnosis of advanced colorectal adenoma or colorectal cancer of the present invention includes:
(i) a step A of measuring a methylation level of CpG present in a region from +1 to +300 relative to a transcription start site of a somatostatin (SST) gene in DNA extracted from feces collected from a subject;
(ii) a step B of measuring a quantitative level of a predetermined internal control gene in DNA extracted from feces collected from the subject; and
(iii) a step C of calculating a normalized methylation level by normalizing the methylation level of CpG in the SST gene measured in the step A by the quantitative level of the predetermined internal control gene measured in the step B; and
(iv) a step D of performing a fecal occult blood test and determining whether the test is positive or negative based on a predetermined cutoff value, in which multivariate analysis is performed using, as indicators, the methylation level measured in the step A and/or the normalized methylation level calculated in the step C, and a result of the fecal occult blood test determined in the step D. The method is not particularly limited as long as it assists in the diagnosis of advanced colorectal adenoma or colorectal cancer in this manner. Hereinafter, the method may also be referred to as the "method A for assisting in the diagnosis of advanced colorectal adenoma or colorectal cancer of the present invention".

Further, a method B for assisting in the diagnosis of advanced colorectal adenoma or colorectal cancer of the present invention includes:
(i) a step A of measuring a methylation level of CpG present in a region from +1 to +300 relative to a transcription start site of a somatostatin (SST) gene in DNA extracted from feces collected from a subject;
(ii) a step B of measuring a quantitative level of a predetermined internal control gene in DNA extracted from feces collected from the subject;
(iii) a step C of calculating a normalized methylation level by normalizing the methylation level of CpG in the SST gene measured in the step A by the quantitative level of the predetermined internal control gene measured in the step B;
(iv) a step D of performing a fecal occult blood test and determining whether the test is positive or negative based on a predetermined cutoff value;
(v) a step E of determining whether the methylation level measured in the step A is positive or negative based on a predetermined cutoff value; and
(vi) a step F of determining whether the normalized methylation level calculated in the step C is positive or negative based on a predetermined cutoff value, in which the subject is determined, for the purpose of diagnostic assistance, as having advanced colorectal adenoma or colorectal cancer if a result determined in the step D and at least one of a result determined in the step E and a result determined in the step F, is positive. The method is not particularly limited as long as it assists in the diagnosis of advanced colorectal adenoma or colorectal cancer in this manner. Hereinafter, the method may also be referred to as the "method B for assisting in the diagnosis of advanced colorectal adenoma or colorectal cancer of the present invention".

In the present specification, examples of the subject may include mammals such as humans, monkeys, pigs, dogs, cats, horses, sheep, mice, and rabbits, with humans being preferable. For example, such subjects may be individuals for whom it is unclear whether or not they are afflicted with advanced colorectal adenoma, or individuals for whom it is unclear whether or not they are afflicted with advanced colorectal adenoma or colorectal cancer. Such subjects also may include individuals with a history of advanced colorectal adenoma or colorectal cancer, who subsequently were completely cured of the advanced colorectal adenoma or colorectal cancer, but for whom it is unclear, at the time of the test, whether they are afflicted with advanced colorectal adenoma or colorectal cancer. Further, the subject may be individuals who have received a negative result by the fecal occult blood test.

In the present specification, the term "colorectal cancer" refers to malignant tumors arising from the transformation of normal cells in the large intestine or the adjacent gastrointestinal tract, characterized by indefinite proliferative potential, invasiveness, and metastatic capacity. Examples thereof may include cecal cancer, colon cancer, and rectal cancer, as well as anal cancer. The term "advanced colorectal adenoma" refers to a colorectal tumor exhibiting cellular morphological abnormalities or structural atypia, specifically defined as adenoma with a diameter of 10 mm or more, adenoma containing a histopathological villous or tubulovillous component, or adenoma exhibiting high-grade dysplasia. Conversely, a benign colorectal tumor that lacks indefinite proliferative potential, invasiveness, and metastatic capacity, specifically defined as adenoma with a diameter of less than 10 mm, adenoma lacking a histopathological villous or tubulovillous component, or adenoma not exhibiting high-grade dysplasia, is classified as non-advanced colorectal adenoma and is thereby distinguished from advanced colorectal adenoma.

In the method for assisting in the diagnosis of advanced colorectal adenoma of the present invention, and in the method A or B for assisting in the diagnosis of advanced colorectal adenoma or colorectal cancer of the present invention, the amount of feces is sufficient as long as it is present in an amount that allows measurement of the DNA methylation level; for example, 0.05 to 0.5g, preferably 0.1 to 0.3g.

Examples of a method for extracting DNA from feces may include a phenol extraction method, a phenol/chloroform extraction method, an alkaline dissolution method, and a method using a commercially available DNA extraction reagent.

The "transcription start site" in the present specification refers to the site at which transcription of mRNA is initiated and corresponds to the position of the first nucleotide of the mRNA. In the present specification, the transcription start site is denoted as "+1", and the position one nucleotide upstream of the transcription start site is denoted as "-1". Therefore, "+300" relative to the transcription start site refers to the position 299 nucleotides downstream when the transcription start site is defined as "+1".

In the present specification, the transcription start site of the somatostatin (SST) gene is located at position 187,670,394 on human chromosome 3 according to the position information database (GRCh38/hg38). Accordingly, the region from +1 to +300 relative to the transcription start site in the SST gene corresponds to positions 187,670,095 to 187,670,394 on human chromosome 3 based on the position information provided by the position information database (GRCh38/hg38). The region from +1 to +300 relative to the transcription start site in the SST gene is not limited as long as it is a methylation-rich region (CpG island), and examples of such a region may include a region from +3 to +280, a region from +7 to +268, a region from +10 to +150, a region from +20 to +100, a region from +40 to +90, or a region from +50 to +60 relative to the transcription start site in the SST gene. The region from +7 to +268 relative to the transcription start site in the SST gene is located at positions 187,670,127 to 187,670,388 on human chromosome 3, as shown in SEQ ID NO: 1. Further, cytosine of CpG in the region from +1 to +300 relative to the transcription start site in the SST gene is preferably cytosine of CpG recognized by at least one restriction enzyme selected from the group consisting of HhaI, HpaII, BstUI, BmgT120I, AatII, BssHII, Cfr10I, HaeII, Hin1I, SacII, and VpaK11BI. More preferably, the cytosine is located at position 187,670,340 (corresponding to +55 relative to the transcription start site) or at position 187,670,342 (corresponding to +53 relative to the transcription start site) on human chromosome 3 (the 214th and 216th position, respectively, in SEQ ID NO: 1). The CpG island can be identified, for example, using the following sites (URL: genome.ucsc.edu/cgi-bin/hgc?hgsid=1793514212_1Ch0xAYmu91SbzU4CgcDI4uMtzZ2&db=hg38&c=chr3&l=1876 68840&r=187671840&o=187670126&t=187670388&g=cpgIslandExt&i=CpG%3A+25). Note that the coding sequence for the SST gene is read from 5' to 3' of the complementary strand of genomic DNA.

In the present specification, the "CpG methylation" refers to a state in which a methyl group is added to the carbon at the 5-position of a cytosine (C) residue in a CpG site, which is a dinucleotide sequence in which a guanine (G) base follows a cytosine (C) base in DNA. In the site, "p" represents the phosphodiester bond between cytosine and guanine.

In the present specification, the CpG methylation level can be evaluated based on the number (copy number) of DNA fragments in which cytosine in the CpG is methylated when the DNA fragments are obtained by amplifying a predetermined region of DNA encoding the SST gene. Specifically, when the evaluation is performed by treatment with a predetermined restriction enzyme, examples of the CpG methylation level may include the copy number of DNA fragments per unit volume of the restriction enzyme solution, for example, per 1 µL, the copy number of DNA fragments in the total volume of the restriction enzyme-treated solution, and the copy number of DNA fragments per unit amount of the DNA source sample (e.g., per 1 g of feces).

In the present specification, a method for measuring the CpG methylation level is not particularly limited, but examples thereof may include a method of treating DNA with a methylation-sensitive restriction enzyme and then amplifying a CpG-containing region recognized by the restriction enzyme. Examples of the methylation-sensitive restriction enzyme may include at least one restriction enzyme selected from the group consisting of HhaI (GCG/C), HpaII (C/CGG), BstUI (also called BshI1236I or AccII depending on the manufacturer) (CG/CG), BmgT120I (GG/NCC), AatII (GACGT/C), BssHII (G/CGCGC), Cfr10I (R/CCGGY), HaeII (RGCGC/Y), Hin1I (GR/CGYC), SacII (CCGC/GG), and VpaK11BI(G/GWCC(G)). Two or more of them may be used in combination. Note that, in the above, "N" means A, G, T or C, "R" means A or G, "Y" means C or T, and "W" means A or T. The methylation-sensitive restriction enzyme described above is an enzyme that can cleave any of the CpG sites present in the region from +1 to +300 relative to the transcription start site in the SST gene.

Further, when treating DNA with the methylation-sensitive restriction enzyme described above, the presence of single-stranded DNA in the template DNA allows such single-stranded DNA to escape cleavage by the restriction enzyme. Consequently, the single-stranded DNA is amplified during the subsequent PCR reaction, leading to false positives. To avoid this, it is preferable to add Exonuclease I (ExoI) to remove the single-stranded DNA. Moreover, when treating DNA with the methylation-sensitive restriction enzyme described above, the DNA may be fragmented by additional restriction enzyme that does not cleave the nucleotide sequence within the amplification target region containing the CpG site, from the viewpoint of improving the efficiency of the methylation-sensitive restriction enzyme reaction and PCR amplification.

The restriction enzyme treatment may be performed in accordance with the optimal pH of the restriction enzyme being used, and the temperature and duration may be adjusted as appropriate. Further, as described in the prior application of the present inventors (Japanese Patent Application No. 2023-043439), the temperature conditions may be changed to perform the second-stage enzyme treatment after the first-stage enzyme treatment without releasing the sealed state, such as by opening the lid of the reaction tube. Alternatively, as described in the above Patent literature 2, after the first-stage enzyme treatment, the second-stage enzyme treatment may be performed by opening the lid of the reaction tube, adding an additional restriction enzyme, and then changing the temperature conditions.

As a method for amplifying the CpG-containing region recognized by the restriction enzyme described above, the restriction enzyme-treated DNA may be amplified as a template using a known DNA amplification technique. Examples thereof may include a PCR (polymerase chain reaction) method such as a digital PCR method or a real-time PCR method, and a LAMP (Loop-Mediated Isothermal Amplification) method. The length of the sequence to be amplified may range, for example, from 20 to 300 bases, preferably 30 to 200 bases, and more preferably 50 to 150 bases.

A primer for amplifying the CpG-containing region recognized by the restriction enzyme described above, and a probe for detecting the amplified nucleic acids may be selected according to the methylation-sensitive restriction enzymes employed. A primer set, probe, or labeled product thereof may be those for individually measuring the methylation of each cytosine in CpG sites that are recognized by the restriction enzymes and included in part or all of the region from +1 to +300 relative to the transcription start site of the SST gene. Alternatively, a primer set, probe, or labeled product thereof may be used for collectively measuring all methylation within the CpG-containing region recognized by the restriction enzymes.

For measuring the CpG methylation level, the primer described above may be any primer that amplifies the CpG-containing region recognized by the methylation-sensitive restriction enzyme used. The probe may be any probe that recognizes a nucleotide sequence in the CpG-containing region. Further, when multiple methylation-sensitive restriction enzyme treatments are performed, all CpG sites recognized by the methylation-sensitive restriction enzymes may be amplified using a single primer set, or alternatively, each CpG-containing region may be amplified using primer sets that individually recognize the specific CpG sites.

When using HhaI and BstUI as the methylation-sensitive restriction enzymes and amplifying a 104-bp region spanning from -27 to +77 relative to the transcription start site as the PCR amplification region for methylated SST, suitable examples of the primer, probe, and labeled product thereof may include: a forward primer consisting of the nucleotide sequence shown in SEQ ID NO: 2, or a forward primer consisting of a nucleotide sequence in which one or several nucleotides in the sequence shown in SEQ ID NO: 2 have been substituted, deleted, inserted, or added; a reverse primer consisting of the nucleotide sequence shown in SEQ ID NO: 3, or a reverse primer consisting of a nucleotide sequence in which one or several nucleotides in the sequence shown in SEQ ID NO: 3 have been substituted, deleted, inserted, or added; a probe consisting of the nucleotide sequence shown in SEQ ID NO: 4, or a probe consisting of a nucleotide sequence in which one or several nucleotides in the sequence shown in SEQ ID NO: 4 have been substituted, deleted, inserted, or added; and labeled products thereof. The above expression "nucleotide sequence in which one or several nucleotides have been substituted, deleted, inserted, or added" refers to a nucleotide sequence in which any number of nucleotides, for example, 1 to 5, preferably 1 to 4, more preferably 1 to 3, even more preferably 1 or 2, and most preferably 1, has been substituted, deleted, inserted, or added.

By using the primer set described above, it is possible to amplify a region encompassing both the cytosine at position 187,670,340 (located at +55 relative to the transcription start site of the SST gene) on human chromosome 3, which is recognized by HhaI, and the cytosine at position 187,670,342 (located at +53 relative to the transcription start site of the SST gene), which is recognized by BstUI. Further, by using the probe shown in SEQ ID NO: 4 described above, it is possible to detect DNA in which both the cytosine at position 187,670,340 (located at +55 relative to the transcription start site of the SST gene) on human chromosome 3, which is recognized by HhaI, and the cytosine at position 187,670,342 (located at +53 relative to the transcription start site of the SST gene), which is recognized by Bst UI, are methylated.

Examples of a labeling substance for the forward primer labeled product, reverse primer labeled product, and probe labeled product may include: an enzyme such as peroxidase (e.g., horseradish peroxidase), alkaline phosphatase, b-D-galactosidase, glucose oxidase, glucose-6-phosphate dehydrogenase, alcohol dehydrogenase, malate dehydrogenase, penicillinase, catalase, apoglucose oxidase, urease, luciferase, or acetylcholinesterase; a fluorescent substance such as fluorescein isothiocyanate, phycobiliprotein, a rare earth metal chelate, dansyl chloride, or tetramethylrhodamine isothiocyanate; a fluorescent protein such as GFP (Green Fluorescent Protein), CFP (Cyan Fluorescent Protein), BFP (Blue Fluorescent Protein), YFP (Yellow Fluorescent Protein), RFP (Red Fluorescent Protein), or luciferase; a radioisotope such as ³H, ¹⁴C, ¹²⁵I, or ¹³¹I; biotin, avidin, or a chemiluminescent substance; and a combination of a reporter fluorescent substance with a quencher fluorescent substance or structure (e.g., a combination of 5-FAM [5-carboxyfluorescein] and 5-TAMRA [5-carboxytetramethylrhodamine], a combination of VIC and MGB [Minor Groove Binder], or a combination of 5-FAM and MGB).

The above step of "measuring the CpG methylation level" may involve not only measuring the CpG methylation in the amplified region from +1 to +300 relative to the transcription start site of the SST gene, but also measuring the CpG methylation in the corresponding region of its complementary strand.

In the method for measuring the CpG methylation level described above, it is preferable without a sodium bisulfite treatment (bisulfite treatment). When the bisulfite treatment is performed, DNA is converted into single-stranded form, rendering it susceptible to degradation. Consequently, approximately 90% of DNA undergoes denaturation and is lost. Therefore, to meet the detection limit of quantitative PCR, which stands at approximately 10 copies, it is necessary to have a starting quantity of at least 100 copies of the target DNA in the DNA sample prior to the bisulfite treatment. Further, when performing absolute quantification analysis of the copy numbers in the quantitative PCR, it is necessary to prepare a calibration curve using a standard substance. However, there is a problem in that the reliability of measured values at low copy numbers is extremely low. In contrast, digital PCR does not require the preparation of a calibration curve, thereby enabling accurate absolute quantification even at low copy numbers.

In the present specification, an internal control gene may be any gene present in two copies per cell. Examples thereof may include the human TERT gene and the human RNase P gene. Examples of the quantitative level of an internal control gene may include the copy number of the internal control gene, the Tt value in the case of amplification by the LAMP method, and the cycle threshold (Ct or Cq value) in the quantitative PCR.

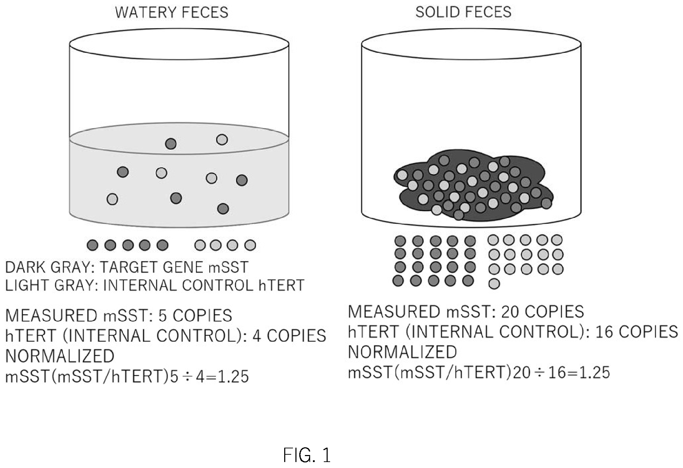

In the present specification, the normalized methylation level can be determined by normalizing the methylation level of CpG in the SST gene measured in the step A by the quantitative level of the predetermined internal control gene measured in the step B. The normalization described above may be performed by any method that enables determination of the methylation level of CpG in the SST gene measured in the step A relative to the quantitative level of the predetermined internal control gene. Preferably, the normalized methylation level described above can be obtained by dividing the methylation level of CpG in the SST gene measured in the step A by the quantitative level of the predetermined internal control gene measured in the step B. The methylated SST value is affected by the amount and conditions of the feces at the time of feces collection. However, by normalizing, preferably dividing, by the quantitative level of the internal control gene, evaluation becomes possible regardless of the conditions or amount of the feces specimen (e.g., watery feces or solid feces) (see FIG. 1 below).

While there are no particular limitations on the method for measuring the quantitative level of an internal control gene, one such a method involves amplifying a partial region of the internal control gene. Amplification of a partial region of the internal control gene is preferably performed in the same reaction tube as that used for measurement of the CpG methylation level described above, from the viewpoints of preventing a decrease in test accuracy due to the difference in the amount of DNA in the tube and improving work efficiency. Further, when the quantitative level of the internal control gene is measured in the same reaction tube as that used for measurement of the CpG methylation level as described above, the partial region of the internal control gene to be amplified is preferably a region that does not include a nucleotide sequence recognized by the methylation-sensitive restriction enzyme present in the reaction tube.

When measuring the quantitative level of the hTERT gene as the internal control gene in the same reaction tube used to measure the CpG methylation level, and when the reaction tube contains HhaI and BstUI as methylation-sensitive restriction enzymes, suitable examples of the primer for amplifying a partial region of hTERT, the probe, and the labeled product may include: a forward primer consisting of the nucleotide sequence shown in SEQ ID NO: 6, or a forward primer consisting of a nucleotide sequence derived from the nucleotide sequence shown in SEQ ID NO: 6 in which one or more nucleotides are substituted, deleted, inserted, or added; a reverse primer consisting of the nucleotide sequence shown in SEQ ID NO: 7, or a reverse primer consisting of a nucleotide sequence derived from the nucleotide sequence shown in SEQ ID NO: 7 in which one or more nucleotides are substituted, deleted, inserted, or added; a probe consisting of the nucleotide sequence shown in SEQ ID NO: 8, or a probe consisting of a nucleotide sequence derived from the nucleotide sequence shown in SEQ ID NO: 8 in which one or more nucleotides are substituted, deleted, inserted, or added; and labeled products thereof.

A predetermined cutoff value in the method for assisting in the diagnosis of advanced colorectal adenoma of the present invention may be set to maximize both sensitivity and specificity. For example, the predetermined cutoff value may be calculated by generating a ROC (Receiver Operating Characteristic) curve using statistical analysis software based on the "normalized methylation level calculated in the step C for DNA extracted from feces collected from patients with advanced colorectal adenoma" and the "normalized methylation level calculated in the step C for DNA extracted from feces collected from control (subjects in whom no tumor is detected via colonoscopy), patients with non-advanced colorectal adenoma, and/or patients with colorectal cancer, preferably, from control, patients with non-advanced colorectal adenoma, and patients with colorectal cancer", and subsequently calculating the value using the Youden Index. Alternatively, the predetermined cutoff value may be calculated based on a mean value of each methylation level, the mean value ± standard deviation (SD), the mean value ± 2SD, the mean value ± 3SD, a median value, the median 25th percentile value, or the median 75th percentile value.

A predetermined cutoff value for the fecal occult blood test used in the step D of the method A or the method B for assisting in the diagnosis of least one of advanced colorectal adenoma and colorectal cancer may be set to maximize both sensitivity and specificity. For example, the predetermined cutoff value may be calculated by generating a ROC curve using statistical analysis software based on the "hemoglobin concentration (ng/mL) in feces specimens measured by the fecal occult blood test in patients with advanced colorectal adenoma and/or patients with colorectal cancer" and the "hemoglobin concentration (ng/mL) in feces specimens measured by the fecal occult blood test in control and/or patients with non-advanced colorectal adenoma", and subsequently calculating the value using the Youden Index. Alternatively, the predetermined cutoff value may be calculated based on a mean value of the hemoglobin concentration in each patient group, the mean value ± standard deviation (SD), the mean value ± 2SD, the mean value ± 3SD, a median value, the median 25th percentile value, or the median 75th percentile value. If the measured value is equal to or greater than the cutoff value, the subject can be determined to be positive for "advanced colorectal adenoma or colorectal cancer". If the measured value is less than the cutoff value, the subject can be determined to be negative for "advanced colorectal adenoma or colorectal cancer".

The cutoff value for the fecal occult blood test in the method A or B for assisting in the diagnosis of advanced colorectal adenoma or colorectal cancer may be set based on values reported in the conventional literatures. Specific examples thereof may include hemoglobin concentrations of 100 ng/ml and 50 ng/ml.

A predetermined cutoff value in the step E of the method B for assisting in the diagnosis of advanced colorectal adenoma or colorectal cancer may be set to maximize both sensitivity and specificity. For example, the predetermined cutoff value may be calculated by generating a ROC curve using statistical analysis software based on the "CpG methylation level measured in the step A in DNA extracted from feces collected from patients with advanced colorectal adenoma and/or patients with colorectal cancer" and the "CpG methylation level measured in the step A in DNA extracted from feces collected from control and/or patients with non-advanced colorectal adenoma", and subsequently calculating the value using the Youden Index. Alternatively, the predetermined cutoff value may be calculated based on a mean value of each methylation level, the mean value ± standard deviation (SD), the mean value ± 2SD, the mean value ± 3SD, a median value, the median 25th percentile value, or the median 75th percentile value. If the measured value is equal to or greater than the cutoff value, the subject can be determined to be positive for "advanced colorectal adenoma or colorectal cancer", and if the measured value is less than the cutoff value, the subject can be determined to be negative for "advanced colorectal adenoma or colorectal cancer".

Further, a predetermined cutoff value in the step F of the method B for assisting in the diagnosis of advanced colorectal adenoma or colorectal cancer may be set to maximize both sensitivity and specificity. For example, the predetermined cutoff value may be calculated by generating a ROC curve using statistical analysis software based on the "normalized methylation level calculated in the step C in DNA extracted from feces collected from patients with advanced colorectal adenoma and/or patients with colorectal cancer" and the "normalized methylation level calculated in the step C in DNA extracted from feces collected from control and/or patients with non-advanced colorectal adenoma", and subsequently calculating the value using the Youden Index. Alternatively, the predetermined cutoff value may be calculated based on a mean value of each normalized methylation level, the mean value ± standard deviation (SD), the mean value ± 2SD, the mean value ± 3SD, a median value, the median 25th percentile value, or the median 75th percentile value. If the measured value is equal to or greater than the cutoff value, the subject can be determined to be positive for "advanced colorectal adenoma or colorectal cancer", and if the measured value is less than the cutoff value, the subject can be determined to be negative for "advanced colorectal adenoma or colorectal cancer".

In the present specification, examples of the multivariate analysis may include logistic regression analysis, linear discriminant analysis, and multiple regression analysis. Further, for continuous variables, the Mann-Whitney U test can be used for comparisons between two groups, while the Kruskal-Wallis test and Dunn's test can be used for multiple comparisons. For categorical variables, the chi-square test and Fisher's exact test can be used. The indicators used for multivariate analysis are not particularly limited, provided that they include the methylation level measured in the step A and/or the normalized methylation level calculated in the step C, and the fecal occult blood test result. Additionally, such indicators may include age and gender. Further, the methylation level measured in the step A and/or the normalized methylation level calculated in the step C may be subjected to logarithmic transformation or power transformation and analyzed using values normalized to a normal distribution. Alternatively, they may be analyzed using values binarized based on a predetermined cutoff value or a predetermined rule. Note that "the results of methylation level measured in the step A and/or the normalized methylation level calculated in the step C, and the results of the fecal occult blood test determined in the step D" may be the results of methylation level measured in the step A and the result of the fecal occult blood test determined in the step D, the results of the normalized methylation level calculated in the step C and the result of the fecal occult blood test determined in the step D, or the results of methylation level measured in the step A, the normalized methylation level calculated in the step C, and the result of the fecal occult blood test determined in the step D. The fecal occult blood test result may be represented as a continuous variable or as a ranked value such as a binarized or ternarized value. When the methylation level (mSST value) is equal to or greater than the predetermined cutoff value, the result may be concluded as mSST-positive, and when the normalized mSST value is equal to or greater than the predetermined cutoff value, the result may be concluded as normalized mSST-positive. Hereinafter, a value is obtained by binarizing the results such that a value of 1 is assigned when either the mSST value or the normalized mSST value, or both, are positive based on the predetermined cutoff value, and a value of 0 is assigned when both are negative, and such a value is referred to as a "binarized mSST value + normalized mSST value".

When performing multivariate analysis using logistic regression analysis, for example, a prediction model equation for calculating a predicted value (P) is constructed by logistic regression analysis in which the presence or absence of a disease is used as a dependent variable and at least two variables including either the normalized mSST value or the binarized mSST value + normalized mSST value described above, and the fecal occult blood test result are used as independent variables. The usefulness of the prediction model equation for assisting in the diagnosis of advanced colorectal adenoma or colorectal cancer is then evaluated. Subsequently, in the prediction model equation determined to be effective based on the verification results, the variables are substituted into the corresponding independent variable terms used for constructing the model equation to calculate a predicted value. Diagnosis of the presence or absence of a disease can be assisted based on the predicted value. Specifically, when the predicted value is calculated based on the normalized methylation level calculated in the step C, the fecal occult blood test result, age, and gender, for example, a prediction model equation is constructed based on the following Equation (I) by logistic regression analysis in which the dependent variable is defined by the presence or absence of least one of advanced colorectal adenoma and colorectal cancer and the independent variables are defined by the normalized mSST value calculated in the step C, the fecal occult blood test result, age, and gender. The usefulness of the prediction model equation for assisting in the diagnosis of advanced colorectal adenoma or colorectal cancer is then evaluated based on a value of the area under the curve (AUC) obtained by ROC curve analysis using the predicted values derived from the model equation. Subsequently, the normalized mSST value, the fecal occult blood test result, age, and gender obtained from a subject are substituted into the prediction model equation determined to be effective based on the verification results, thereby calculating the predicted value p. When the predicted value p is calculated based on the binarized mSST value + normalized mSST value, the fecal occult blood test result, and age, for example, a prediction model equation may be constructed based on Equation (II) instead of Equation (I), and the predicted value p may be calculated based on the prediction model equation.
[Mathematical 1] $p = \frac{1}{1 + {e}^{- \left\{{β}_{0}+{β}_{1}\left(normalized mSST value\right)+{β}_{2}\left(fecal occult blood test result\right)+{β}_{3}\left(age\right)+{β}_{4}\left(gender\right)\right\}}}$ Equation (I)

In the above Equation (I), "β0" represents an intercept (constant term), and "β1-β4" represent regression coefficients. The calculated value of the normalized mSST is substituted for the "normalized mSST value", a value of 1 is substituted for a positive "fecal occult blood test result" and 0 for a negative "fecal occult blood test result", the subject's age is substituted for "age", and a value of 1 is substituted for male and 0 for female for "gender".
[Mathematical 2] $p = \frac{1}{1 + {e}^{- \left\{{β}_{0}+{β}_{1}\left(mSST value+normalized mSST value\right)+{β}_{2}\left(fecal occult blood test result\right)+{β}_{3}\left(age\right)\right\}}}$ Equation (II)

In the above Equation (II), "β0" represents an intercept (constant term), and "β1-β3" represent regression coefficients. The term "(mSST value + normalized mSST value)" represents the binarized mSST value + normalized mSST value, in which a value of 1 is substituted when either the mSST value or the normalized mSST value, or both, are positive, and a value of 0 is substituted when both are negative. For the "fecal occult blood test result", a value of 1 is substituted for a positive result and 0 for a negative result, and the subject's age is substituted for "age".

In the method for assisting in the diagnosis of advanced colorectal adenoma of the present invention, the diagnosis of advanced colorectal adenoma may be performed by comparing a normalized methylation level with a predetermined cutoff value, the normalized methylation level being calculated by normalizing, preferably dividing, the methylation level of CpG in the SST gene measured in the step A by the quantitative level of the predetermined internal control gene measured in the step B. If the normalized methylation level is equal to or greater than the predetermined cutoff value, the subject can be evaluated as having a high likelihood of suffering from advanced colorectal adenoma. If the normalized methylation level is less than the predetermined cutoff value, the subject can be evaluated as having a low likelihood of suffering from advanced colorectal adenoma.

In the method A for assisting in the diagnosis of advanced colorectal adenoma or colorectal cancer of the present invention, the diagnosis of advanced colorectal adenoma or colorectal cancer can be performed based on a value (predicted value) obtained by the multivariate analysis described above. Specifically, the closer the predicted value is to 1, the higher the likelihood that the "subject suffers from advanced colorectal adenoma or colorectal cancer", whereas the closer the predicted value is to 0, the lower the likelihood that the "subject suffers from advanced colorectal adenoma or colorectal cancer". Thus, based on the value (predicted value), it is possible to diagnose whether the subject suffers from advanced colorectal adenoma or colorectal cancer.

Further, based on the predicted values calculated from the prediction model equation constructed based on the above Equation (I) or (II), a cutoff value for determining whether a "subject suffers from advanced colorectal adenoma or colorectal cancer" can be determined. If the predicted value is higher than the cutoff value, the subject can be evaluated as having a high likelihood of suffering from advanced colorectal adenoma or colorectal cancer, whereas if the predicted value is equal to or less than the cutoff value, the subject can be evaluated as having a low likelihood of suffering from advanced colorectal adenoma or colorectal cancer.

In addition, based on the predicted values calculated from the above Equations (I) or (II), a cutoff value X of the predicted value for distinguishing control and non-advanced adenoma from advanced adenoma can be calculated, and a cutoff value Y for distinguishing advanced adenoma from colorectal cancer can be calculated based on the predicted values of the advanced adenoma group and the colorectal cancer group. If the predicted value for a subject calculated from the above Equation (I) or (II) is less than the cutoff value X, the subject can be evaluated as having a high likelihood of being control or having non-advanced adenoma, or as having a high likelihood of not suffering from advanced adenoma or colorectal cancer. If the predicted value is equal to or greater than the cutoff value X but less than the cutoff value Y, the subject can be evaluated as having a high likelihood of suffering from advanced adenoma. If the predicted value is equal to or greater than the cutoff value Y, the subject can be evaluated as having a high likelihood of suffering from colorectal cancer. The cutoff value may be determined, for example, by generating a ROC curve using statistical analysis software based on the predicted values calculated from the above Equation (I) or (II) using the independent variables obtained from subjects in the control group, the non-advanced adenoma group, the advanced adenoma group, and the colorectal cancer group, and calculating the cutoff value using the Youden index. Alternatively, the cutoff value may be calculated based on a mean of the predicted values for each group, the mean ± standard deviation (SD), the mean ± 2SD, the mean ± 3SD, a median, the median 25th percentile value, or the median 75th percentile value. In other words, in the method A for assisting in the diagnosis of advanced colorectal adenoma or colorectal cancer of the present invention, by setting the cutoff values X and Y, the method may also be regarded as a "method for assisting in the diagnosis of advanced colorectal adenoma".

In the method B for assisting in the diagnosis of advanced colorectal adenoma or colorectal cancer of the present invention, if the "result of the fecal occult blood test determined in the step D", or the "methylation level of CpG in the SST gene determined in the step E and/or the normalized methylation level calculated in the step F", is positive, the subject can be determined, for the purpose of diagnostic assistance, as having advanced colorectal adenoma or colorectal cancer. On the other hand, if both the "result of the fecal occult blood test determined in the step D" and the "methylation level of CpG in the SST gene determined in the step E and/or the normalized methylation level calculated in the step F" are negative, the subject can be determined, for the purpose of diagnostic assistance, as not having advanced colorectal adenoma or colorectal cancer.

In the method B for assisting in the diagnosis of advanced colorectal adenoma or colorectal cancer of the present invention, if both the result determined in the step D and at least one of the result determined in the steps E and a result determined in the step F are positive, the subject may be determined, for the purpose of diagnostic assistance, as having advanced colorectal adenoma or colorectal cancer.

Hereinafter, the present invention is described in more detail with reference to Examples. However, the technical scope of the present invention is not limited to these illustrative examples.

Seventy-nine individuals in whom no tumor was detected by colonoscopy were assigned to the control group. The study included 43 patients with non-advanced colorectal adenoma, 117 patients with advanced colorectal adenoma, and 126 patients with colorectal cancer (stages I-IV according to the TNM classification, 8th edition, UICC). Feces specimens for the fecal occult blood test were collected using an S fecal collection container (Eiken Chemical Co., Ltd.). Feces specimens for methylation analysis of DNA extracted from feces (fecal DNA test) were collected using a fecal collection cup. In the following Examples, advanced colorectal adenoma may also be referred to simply as "advanced adenoma", and non-advanced colorectal adenoma may also be referred to simply as "non-advanced adenoma".

### (1) Fecal occult blood test

Feces specimens collected using the S fecal collection container were used. The hemoglobin concentration in the feces specimens was measured using OC-Hemodia (registered trademark) Auto S "Eiken" (Eiken Chemical Co., Ltd.) as the measurement reagent and an OC-Sensor io (registered trademark) (Eiken Chemical Co., Ltd.) as the measurement instrument. The cutoff value for the fecal occult blood test was set at a hemoglobin concentration of 100 ng/mL, which is commonly used. A value equal to or greater than the cutoff value was determined to be positive for the fecal occult blood test (reference for the fecal occult blood test cutoff: Ministry of Health, Labour and Welfare website, www.mhlw.go.jp/shingi/2007/06/dl/s0626-13i_0002.pdf, accessed Oct. 2, 2023).

### (2) Fecal DNA test

Approximately 200 mg of feces was collected from the feces specimen in the fecal collection cup, and DNA was extracted using the QIAamp Fast DNA Stool Mini Kit (Qiagen) according to the protocol provided with the kit. The copy number of methylated SST as a target gene and the copy number of the hTERT gene as an internal control gene were measured using an improved version of a highly sensitive methylation analysis method developed by the present inventors, namely the CORD assay (combined restriction enzymes and digital PCR; see the above Non-patent literature 3 and Japanese Patent Application No. 2023-043439). In this CORD assay, methylation-sensitive restriction enzymes are added to the feces-derived DNA and incubated so that unmethylated DNA is cleaved while methylated DNA remains uncleaved. In digital PCR, only the methylated DNA that has not been cleaved is amplified, thereby enabling analysis of methylation by measuring the copy number of the amplified DNA.

The improved highly sensitive methylation analysis method is described below.
1. Add 10 µL of a DNA sample to a 1.5 mL tube.
2. Add 1 µL of AmpliTaq Gold Buffer II (AmpliTaq Gold DNA Polymerase Kit, Thermo Fisher Scientific Inc.).
3. Add 1 µL of 25 mM MgCl2 solution (N808-0241; AmpliTaq Gold DNA Polymerase Kit, Thermo Fisher Scientific Inc.).
4. Add 1 µL (10 U) of HhaI (Thermo Fisher Scientific Inc.).
5. Add 1 µL (10 U) of HpaII (Thermo Fisher Scientific Inc.).
6. Add 1 µL (20 U) of ExoI (Thermo Fisher Scientific Inc.).
7. Add 1 µL (10 U) of BstUI (New England BioLabs).
8. Mix by pipetting and close the lid of the tube to seal it.
9. Incubate at 37°C for 1 hour.
10. Incubate at 60°C for 16 hours.
11. Incubate at 98°C for 10 minutes.

HhaI and BstUI described above are methylation-sensitive restriction enzymes that recognize and cleave the nucleotide sequences 5'-GCGC-3' and 5'-CGCG-3', respectively. Because these nucleotide sequences remain uncleaved when they are methylated, they can subsequently be amplified by PCR. Accordingly, methylation of cytosines at positions 187,670,340 (+55 relative to the transcription start site) and 187,670,342 (+53 relative to the transcription start site) on human chromosome 3 can be analyzed. Note that HpaII was used for DNA fragmentation.

A digital PCR system ,QX200 Droplet Digital PCR System (Bio-Rad Laboratories, Inc.) was used.

After completion of the enzymatic reaction, 2 µL of the enzyme-treated DNA solution treated under the above conditions, 8 µL of water, and 12 µL of PCR reaction solution were mixed to prepare a DNA-containing PCR reaction solution. The PCR reaction solution includes 2 µL of a primer/probe mix and 10 µL of 2× ddPCR master mix (Bio-Rad Laboratories, Inc.). The primer/probe mix includes a 10 µM forward primer, a 10 µM reverse primer, and a 10 µM probe for amplifying a predetermined region of the SST gene and measuring the methylation level of predetermined cytosine therein, and a 5 µM forward primer, a 5 µM reverse primer, and a 2.5 µM probe for amplifying a predetermined region of the hTERT gene. Multiplex PCR was performed using the DNA-containing PCR reaction solution described above.

The nucleotide sequences of the primers for amplifying the region of human chromosome 3 from positions 187,670,318 to 187,670,421, and the nucleotide sequence of the probe for detecting the region of human chromosome 3 at positions 187,670,340 (+55 relative to the transcription start site) and 187,670,342 (+53 relative to the transcription start site) are as follows. The above primers amplify the nucleotide sequence shown in SEQ ID NO: 5. Further, the probe used is nucleic acids having the nucleotide sequence shown in SEQ ID NO: 4, to which a fluorescent dye FAM is attached at the 5' end and a quencher BHQ1 is attached at the 3' end.
Forward primer: 5'-GAGTGGCTGGTCAAACTCTA-3' (SEQ ID NO: 2)
Reverse primer: 5'-AAAACAGAGGGAGACGGTTG-3' (SEQ ID NO: 3)
Probe: 5'-FAM-TGCTGCCTGCTGATCCGCGCC-BHQ1-3' (SEQ ID NO: 4)

Further, the nucleotide sequences of primers for amplifying the human TERT gene as an internal control gene and of a probe for detecting the same are as follows. The above primers amplify the nucleotide sequence shown in SEQ ID NO: 9. The probe used is a polynucleotide having the nucleotide sequence shown in SEQ ID NO: 8, to which a fluorescent dye VIC is attached at the 5' end and a quencher MGB is attached at the 3' end. The nucleotide sequence shown in SEQ ID NO: 9 to be amplified does not include nucleotide sequences recognized by the methylation-sensitive restriction enzymes HhaI, BstUI, and HpaII.
Forward primer: 5'-GGGTCCTCGCCTGTGTACAG-3' (SEQ ID NO: 6)
Reverse primer: 5'-CCTGGGAGCTCTGGGAATTT-3' (SEQ ID NO: 7)
Probe: 5'-VIC-CACACCTTTGGTCACTC-MGB-3' (SEQ ID NO: 8)

Table 1 shows the nucleotide sequences of the primers, probes, and PCR amplification products. In Table 1, within the nucleotide sequence column of the PCR amplification product for SST (lower row), the bolded "C" among the bases indicates either a cytosine at position 187,670,340 (+55 relative to the transcription start site) on human chromosome 3, which is a potential cleavage site (GCG/C) for the restriction enzyme HhaI, or a cytosine at position 187,670,342 (+53 relative to the transcription start site), which is a potential cleavage site (CG/CG) for BstUI. Note that since these restriction enzymes are methylation-sensitive, the sites are not cleaved if the cytosine (C) therein is methylated.

**[Table 1]**

| Genes | Probes(5'-3') | Forward primers(5'-3') | Reverse primers(5'-3') | PCR amplification product nucleotide sequences(5'-3') Bold "C" indicates cytosine targeted for methylation modification | PCR amplification product position information (PCR amplification product length bps) (Database GRCh38/hg38) |
|---|---|---|---|---|---|
| TERT Internal control | VIC- CACACCTTTGGT CACTC-MGB (SEQ ID NO: 8) | GGGTCCTCGCCT GTGTACAG (SEQ ID NO: 6) | CCTGGGAGCTCT GGGAATTT (SEQ ID NO: 7) | GGGTCCTCGCCTGTGTACAG GGCACACCTTTGGTCACTCCA AATTCCCAGAGCTCCCAGG (SEQ ID NO: 9) | chr5:1,253,260-1,253,319 (60bp) |
| SST | FAM- TGCTGCCTGCT GATCCGCGCC- BHQ1 (SEQ ID NO: 4) | GAGTGGCTGGTC AAACTCTA （SEQ ID NO: 2） | AAAACAGAGGGA GACGGTTG (SEQ ID NO: 3) | GAGTGGCTGGTCAAACTCTA GGCGCGGATCAGCAGGCAG CAGCGATGGCTCCGAACCTC GCTCCTAAAGCGGCTTGTGT GCTCTCAACCGTCTCCCTCTG TTTT (SEQ ID NO: 5) | chr3:187,670,318-187,670,421 (104bp) |

Droplets were generated from this DNA-containing PCR reaction solution using an Automated Droplet Generator (Bio-Rad Laboratories, Inc.). Then, using a thermal cycler, the solution was preheated at 95°C for 10 minutes, followed by 40 cycles of DNA amplification including thermal denaturation at 94°C for 30 seconds and annealing at 56°C for 60 seconds. Finally, the solution was heated at 98°C for 10 minutes. Following DNA amplification, QX200 Droplet Reader (Bio-Rad Laboratories, Inc.) and QuantaSoft software (Bio-Rad Laboratories, Inc.) were used to detect the fluorescent dye derived from the TaqMan probe in each droplet. This allowed for the quantification of the number (copy number) of DNA fragments in which the cytosine at the CpG site in the amplified region of the SST gene was methylated, thereby calculating the methylated SST copy number (mSST value). Further, a value obtained by dividing the mSST value by the hTERT copy number (methylated SST copy number/hTERT copy number: hereinafter also referred to as the "normalized mSST value") was calculated.

### (3) Statistical analysis using fecal occult blood test and fecal DNA test as indicators

In the multivariate analysis, statistical analyses were performed to evaluate the relationships between measured or calculated indicators (mSST values, normalized mSST values, binarized mSST + normalized mSST, and binarized fecal occult blood test results) and the clinicopathological background of the subjects. Analyses were performed using GraphPad Prism ver. 9 (GraphPad Software Corp) and StatFlex ver. 7 (Artec Corp). For continuous variables, comparisons between two groups were conducted using the Mann-Whitney U test, while multiple comparisons were performed using the Kruskal-Wallis test and Dunn's test. For categorical variables, the chi-square test and Fisher's exact test were used. Multivariate analysis was performed using multiple logistic regression analysis. For each test marker, ROC analysis was performed to compare the diagnostic performance in distinguishing the "advanced adenoma group" or "at least one of the advanced adenoma group and the colorectal cancer group" from the control group. The area under the curve (AUC) was obtained based on the ROC curve. Statistical significance was defined as p<0.05. Further, cutoff values were set based on measurement values at various specificity levels, and the corresponding sensitivity was calculated.

### (i) Univariate analysis

For the fecal occult blood test, mSST values, and normalized mSST values, the diagnostic performance (sensitivity and specificity) for detecting "advanced adenoma" or "advanced adenoma or colorectal cancer" was evaluated for both individual tests and combined tests. Cutoff values were set as the measurement values corresponding to a specificity of 95% (mSST cutoff: 67.9, normalized mSST cutoff: 0.50). A supplementary explanation of the normalized mSST value is provided in FIG. 1.

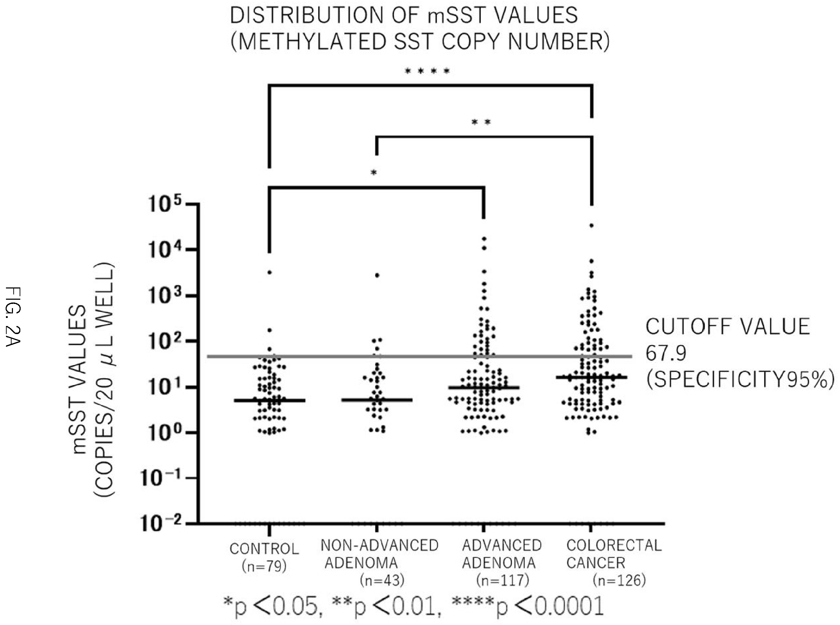

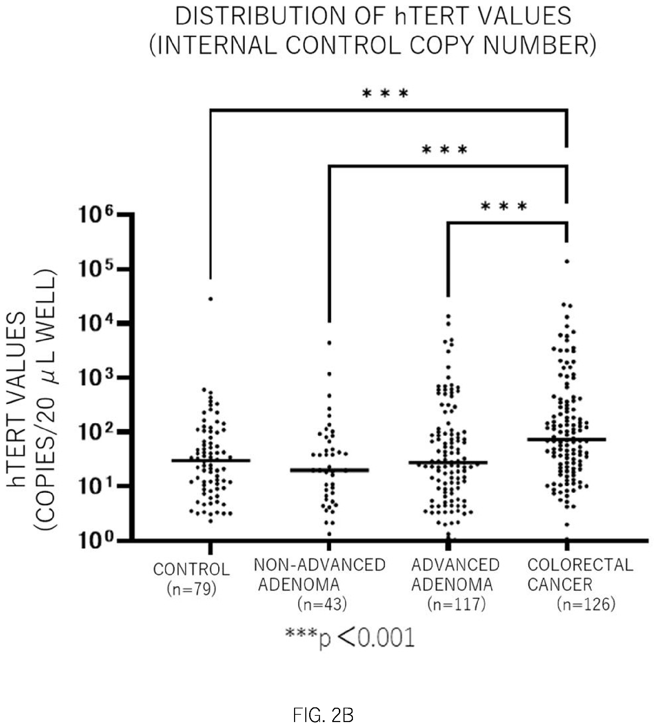

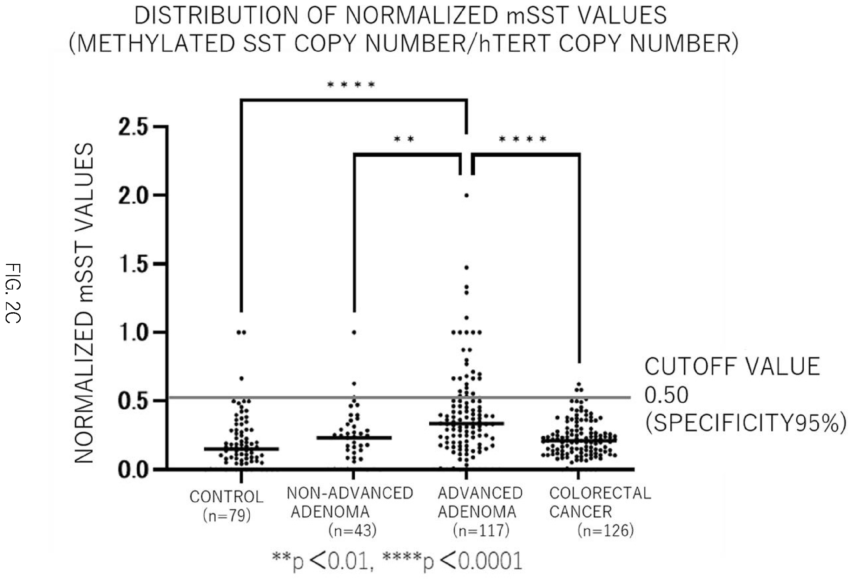

Next, the distributions of mSST values, hTERT values, and normalized mSST values in the control group, non-advanced adenoma group, advanced adenoma group, and colorectal cancer group (stages I-IV) are shown in FIG. 2A, FIG. 2B, and FIG. 2C, respectively. To determine differences in the mSST values and the normalized mSST values among the groups, multiple comparisons were performed using Dunn's test. For the mSST values, the advanced adenoma group showed significantly higher values only in comparison with the control group (FIG. 2A). In contrast, for the normalized mSST values, the advanced adenoma group showed significantly higher values compared to all other groups, including the control group, non-advanced adenoma group, and colorectal cancer group (FIG. 2C). These results demonstrate that the normalized mSST is a biomarker specific to advanced adenoma. In FIGS. 2A, 2B, and 2C, the horizontal bar in each group indicates the median.

Further, the sensitivity and specificity for each group were evaluated under three conditions described above: "fecal occult blood test alone", "fecal DNA test alone", and "combined test of fecal DNA test and fecal occult blood test". The results are shown in Table 2.

**[Table 2]**

| | | Fecal occult blood test alone | | | Feces DNA test alone | | | Combined test | | |
|---|---|---|---|---|---|---|---|---|---|---|
| Group | Total | Number of positives | Sensitivity (%) | 95% Confidence interval(%) | Number of positives | Sensitivity (%) | 95% Confidence interval(%) | Number of positives | Sensitivity (%) | 95% Confidence interval(%) |
| Non-advanced adenoma | 43 | 3 | 7.0 | 2.4 ~ 186 | 6 | 14.0 | 5.3 ~ 27.9 | 9 | 20.9 | 10.0 ~ 36.0 |
| Advanced adenoma | 117 | 34 | 29.1 | 21.6 ~ 37.9 | 46 | 39.3 | 30.5 ~ 48.2 | 72 | 61.5 | 52.7 ~ 70.4 |
| Colorectal cancer | 126 | 115 | 91.3 | 85.0 ~ 95.1 | 38 | 30.2 | 22.1 ~ 38.2 | 119 | 94.4 | 88.9 ~ 97.7 |
| Colorectal cancer + Advanced adenoma | 243 | 149 | 61.3 | 55.1 ~ 67.2 | 84 | 34.6 | 28.6 ~ 40.5 | 191 | 78.6 | 73.4 ~ 83.8 |
| Group | | | Specificity (%) | | | Specificity (%) | | | Specificity (%) | |
| Control | 79 | 10 | 87.3 | 78.2 ~ 93.0 | 6 | 92.4 | 84.2 ~ 97.2 | 16 | 79.7 | 70.9 ~ 88.6 |
| Control + Non-advanced adenoma | 122 | 13 | 89.3 | 82.6 ~ 93.7 | 12 | 90.2 | 84.9 ~ 95.4 | 25 | 79.5 | 72.3 ~ 86.7 |

### (i)-1 Fecal occult blood test alone

The test sensitivity was 7.0% for the non-advanced adenoma group, 29.1% for the advanced adenoma group, 91.3% for the colorectal cancer group, and 61.3% for the advanced adenoma group + colorectal cancer group. Regarding specificity, it was 87.3% when only the control group was used as the reference group, and 89.3% when both the control group and the non-advanced adenoma group (control group + non-advanced adenoma group) were used as the reference group.

### (i)-2 Fecal DNA test alone

For the fecal DNA test, in order to minimize the number of false positives, the cutoff value was defined based on measurement values at a specificity of 95%. Specifically, the test cutoff value was set at 67.9 for the mSST value and at 0.50 for the normalized mSST value. For determination of a positive result in the fecal DNA test, a result was considered positive when either the mSST value or the normalized mSST value was equal to or greater than the cutoff value, or when both were equal to or greater than the cutoff value. The test sensitivity was 14.0% for the non-advanced adenoma group, 39.3% for the advanced adenoma group, 30.2% for the colorectal cancer group, and 34.6% for the advanced adenoma group + the colorectal cancer group. Regarding specificity, it was 92.4% when only the control group was used as the reference group, and 90.2% when the control group + the non-advanced adenoma group was used as the reference group.

### (i)-3 Combined test of fecal DNA test and fecal occult blood test

A "combined test positive" result was defined as a case in which either the fecal occult blood test or the fecal DNA test was positive, or both the fecal occult blood test and the fecal DNA test were positive. The test sensitivity was 20.9% for the non-advanced adenoma group, 61.5% for the advanced adenoma group, 94.4% for the colorectal cancer group, and 78.6% for the advanced adenoma group + the colorectal cancer group. Regarding specificity, it was 79.7% when only the control group was used as the reference group, and 79.5% when the control group + the non-advanced adenoma group was used as the reference group. The test sensitivity for advanced adenoma was 61.5% when the fecal DNA test and the fecal occult blood test were combined, which represented a significant improvement compared to 29.1% in the fecal occult blood test alone described in (i)-1 (Table 2).

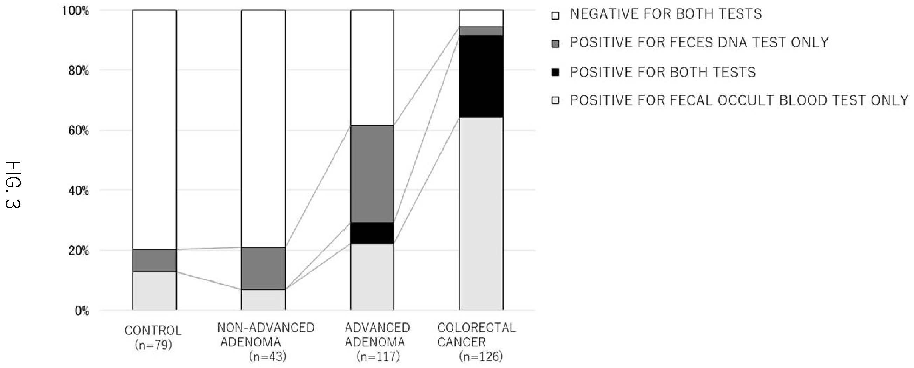

Based on the results shown in Table 2, the data were graphically presented by classifying subjects into the "control group", the "non-advanced adenoma group", the "advanced adenoma group", and the "colorectal cancer group", as shown in FIG. 3. FIG. 3 also confirms that the fecal DNA test facilitates detection of the advanced adenoma group with high sensitivity. In addition, when at least one of the fecal occult blood test and the DNA test is positive, there is a high likelihood that the subject suffers from advanced colorectal adenoma or colorectal cancer. When both the fecal occult blood test and the fecal DNA test are positive (indicated as "Positive for both tests" in FIG. 3), it is found that the likelihood of having advanced adenoma or colorectal cancer is extremely high.

### (ii) Multivariate analysis

In order to identify factors that independently affect the presence or absence of disease, a disease prediction model equation was constructed by multiple logistic regression analysis using the presence or absence of disease as a dependent variable and using the mSST value, the normalized mSST value, the fecal occult blood test result, age, and gender as independent variables. Subsequently, ROC curve analysis was performed using the predicted values derived from the model equation. Based on a value of the area under the curve (AUC) obtained from the ROC curve analysis, it was evaluated whether the prediction model equation was useful as an assistance in the diagnosis of advanced adenoma. In addition, the test sensitivity and specificity for advanced adenoma and colorectal cancer (hereinafter also referred to as "advanced adenoma + colorectal cancer") were evaluated. The construction and validation of the above model equation is described in detail below.

First, the multiple logistic regression analysis was performed using the presence or absence of disease (control + non-advanced adenoma vs. advanced adenoma) as the dependent variable, and using the normalized mSST value, the fecal occult blood test result, age, and gender as independent variables. In the statistical analysis, for qualitative data, the normalized mSST value was adopted as a calculated value, and the fecal occult blood test result and gender were binarized (fecal occult blood test: positive=1, negative=0; gender: male=1, female=0). As a result of the multiple logistic regression analysis, it is found that the normalized mSST value, the fecal occult blood test result, age, and gender are independent prediction factors of advanced adenoma. Subsequently, a prediction model equation combining these independent variables was derived as represented by the following Equation (III).
[Mathematical 3] $\begin{matrix}p = \frac{1}{1 + {e}^{- x}} \\ x = - 7.51 + 1.941 \times normalized mSST value + 0.9911 \times fecal occult blood test result + 0.1009 \times \\ age + 0.8072 \times gender\end{matrix}$ Equation (III)

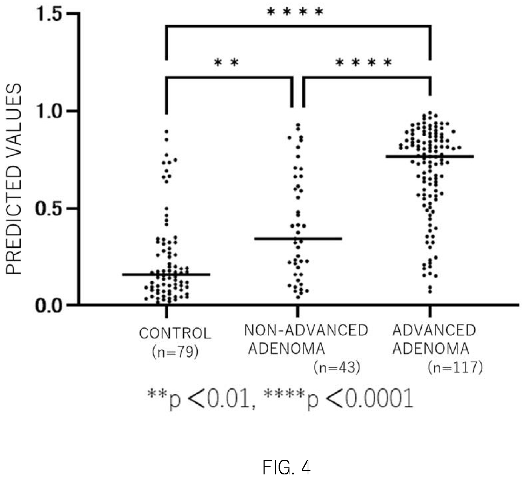

The predicted values were calculated by substituting the normalized mSST value, the fecal occult blood test result, age, and gender into Equation (III) for each of the control group, the non-advanced adenoma group, and the advanced adenoma group. The distribution of the predicted values was plotted, and multiple comparisons of the predicted values among the groups were performed using Dunn's test. The results are shown in FIG. 4. According to the results in FIG. 4, the advanced adenoma group exhibited significantly higher predicted values than those of the control group and the non-advanced adenoma group. In FIG. 4, the horizontal bar for each group represents the median.

The p-value (predicted value) calculated by Equation (III) ranges from 0 to 1, where a value closer to 1 indicates a higher likelihood of advanced adenoma, whereas a value closer to 0 indicates a higher likelihood of belonging to the reference group (control (no tumor) or non-advanced adenoma).

According to the results in FIG. 4, it is found that the predicted values calculated by the above Equation (III) exhibit a statistically significant difference between the control group and the advanced adenoma group, and between the non-advanced adenoma group and the advanced adenoma group.

Next, for the qualitative data, a predictive model equation was constructed using a binarized mSST value + normalized mSST value.

Then, multiple logistic regression analysis was performed using the presence or absence of disease (control + non-advanced adenoma vs. advanced adenoma + colorectal cancer) as the dependent variable, and using the binarized mSST value + normalized mSST value (mSST value + normalized mSST value), the fecal occult blood test result, and age as independent variables. In the statistical analysis, the qualitative data were binarized as follows: (mSST value + normalized mSST value) (positive = 1, negative = 0) and fecal occult blood test result (positive = 1, negative = 0). As a result of the multiple logistic regression analysis, (mSST value + normalized mSST value), the fecal occult blood test result, and age were identified as independent prediction factors of advanced adenoma. Subsequently, a prediction model equation combining these independent variables was derived as represented by the following Equation (IV).
[Mathematical 4] $\begin{matrix}p = \frac{1}{1 + {e}^{- x}} \\ x = - 6.441 + 1.622 \times \left(mSST value+normalized mSST value\right) + 2.69 \times fecal occult blood test \\ result + 0.09609 \times age\end{matrix}$ Equation (IV)

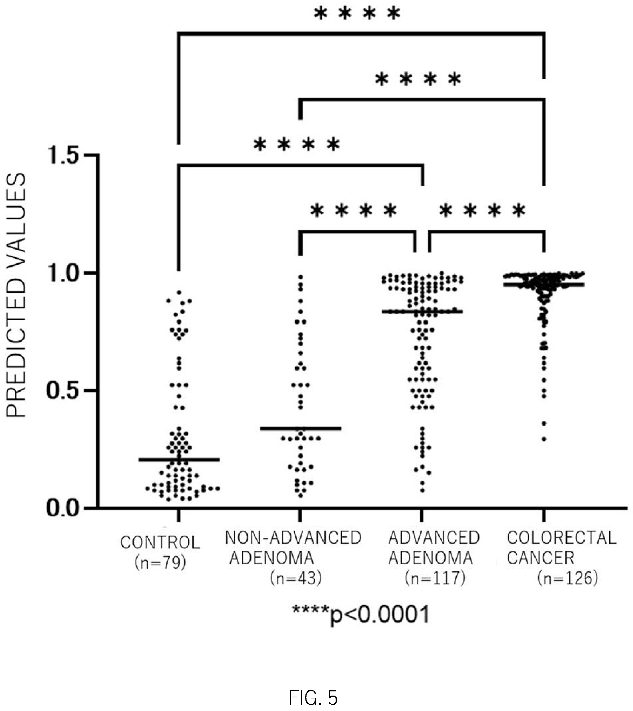

Next, the predicted values were calculated by substituting (mSST value + normalized mSST value), the fecal occult blood test result, and age into the above Equation (IV) for each of the control group, the non-advanced adenoma group, the advanced adenoma group, and the colorectal cancer group. The distribution of the predicted values was plotted, and multiple comparisons of the predicted values based on Equation (IV) among the groups were performed using Dunn's test. The results are shown in FIG. 5. In FIG. 5, the horizontal bar for each group represents the median. The p-value (predicted value) calculated by Equation (IV) ranges from 0 to 1, where a value closer to 1 indicates a higher likelihood of advanced adenoma or colorectal cancer, whereas a value closer to 0 indicates a higher likelihood of belonging to the reference group (no tumor or non-advanced adenoma).

According to the results in FIG. 5, the predicted values calculated by the above Equation (IV) were significantly higher in the advanced adenoma group and the colorectal cancer group than in the control group and the non-advanced adenoma group. Specifically, statistically significant differences in the predicted values were observed between the control group and the advanced adenoma group, and between the non-advanced adenoma group and the advanced adenoma group. In addition, statistically significant differences in the predicted values were observed between the control group and the colorectal cancer group, between the non-advanced adenoma group and the colorectal cancer group, and between the advanced adenoma group and the colorectal cancer group.

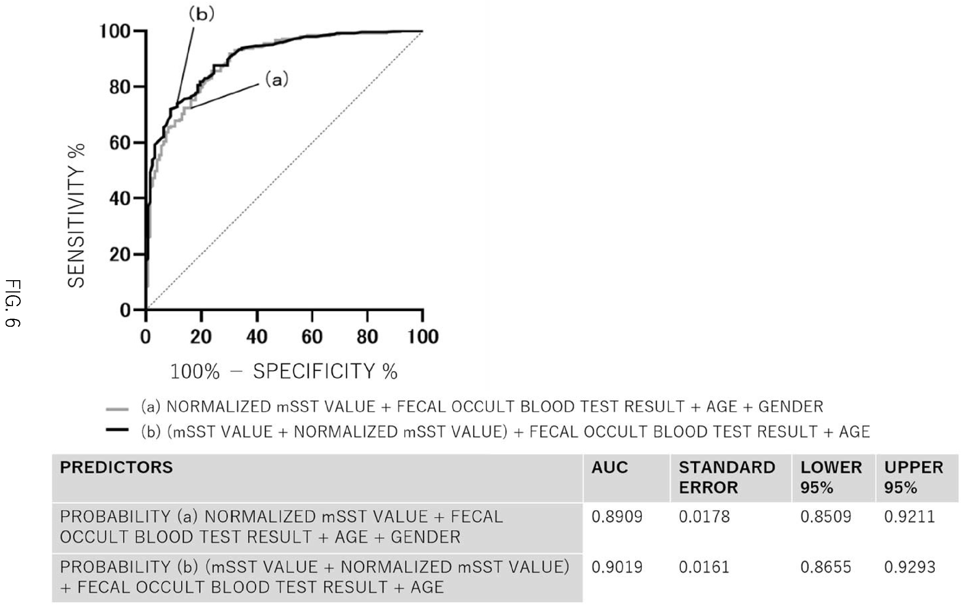

Next, using the ROC analysis (reference group (control group + non-advanced adenoma group) vs. disease group (advanced adenoma group + colorectal cancer group)), the ability of the above prediction Equation (III) and Equation (IV) for distinguishing between the reference group and the disease group was evaluated. The combinations of independent variables were as follows, in two patterns: Equation (III): $\left(a\right) normalized mSST value + fecal occult blood test result + age + gender$ Equation (IV): $\left(b\right) \left(mSST value + normalized mSST value\right) + fecal occult blood test result + age$ The results are shown in FIG. 6.

The AUC obtained by ROC analysis was 0.8909 for the pattern (a) and 0.9019 for the pattern (b), both indicating high values.

Based on the results shown in the above FIGS. 4 to 6, it is found that the p-value (predicted value) calculated by Equation (III) or Equation (IV) can be used to assist in the diagnosis of advanced colorectal adenoma or colorectal cancer.

In addition, as described above, statistically significant differences were also observed between the advanced adenoma group and the colorectal cancer group in the predicted values calculated by the above Equation (IV). Thus, based on the predicted values for each of the control (no tumor) group, the non-advanced adenoma group, and the advanced adenoma group, a cutoff value X of the predicted values for distinguishing the control and non-advanced adenoma from advanced adenoma was calculated. Further, based on the predicted values for each of the advanced adenoma group and the colorectal cancer group, a cutoff value Y for distinguishing advanced adenoma from colorectal cancer was calculated. Once the predicted value based on the above Equation (IV) for a subject is obtained, the subject can be classified as "control or non-advanced adenoma", "advanced adenoma", or "colorectal cancer". Specifically, if the predicted value based on the above Equation (IV) is less than the cutoff value X, the subject is classified as control or non-advanced adenoma, if the predicted value is equal to or greater than the cutoff value X and less than the cutoff value Y, the subject is classified as advanced adenoma, and if the predicted value is equal to or greater than the cutoff value Y, the subject is classified as colorectal cancer, thereby enabling assistance in the diagnosis.

Further, Table 3A shows the test sensitivity for advanced adenoma when the cutoff values were set so as to achieve specificities of 95%, 90%, 85%, and 80% based on ROC analysis. Table 3B shows the test sensitivity for advanced adenoma and colorectal cancer when the cutoff values were set so as to achieve specificities of 95%, 90%, 85%, and 80%. The test sensitivity for advanced adenoma was 42.7%, 56.4%, 61.5%, and 68.4%, respectively. The test sensitivity for advanced adenoma + colorectal cancer was 61.7%, 72.0%, 75.7%, and 81.9%, respectively. For the results in Table 3A, a prediction model equation was derived by setting the dependent variable as the presence or absence of disease (control + non-advanced adenoma vs. advanced adenoma), with the independent variables being the same as those used to derive Equation (IV). The predicted values were then calculated by substituting each variable into the corresponding independent variable term of the prediction model equation, thereby determining the test sensitivity. Also, for the results in Table 3B, the dependent variable was defined as the presence or absence of disease (control + non-advanced adenoma vs. advanced adenoma + colorectal cancer). The predicted values were then calculated by substituting each variable into the corresponding independent variable term of the model Equation (IV), thereby determining the test sensitivity.

**[Table 3A]**

| | | Control + Non-advanced adenoma vs. Advanced adenoma | | |
|---|---|---|---|---|
| | | Cutoff | Sensitivity(%) | 95% Confidence interval (%) |
| Specificity(%) | 95 | 0.87 | 42.7 | 33.8 ~ 51.7 |
| | 90 | 0.79 | 56.4 | 47.4 ~ 65.4 |
| | 85 | 0.74 | 61.5 | 52.7 ~ 70.4 |
| | 80 | 0.64 | 68.4 | 60.0 ~ 76.8 |
| AUC | | 0.85 | | |

**[Table 3B]**

| | | Control + Non-advanced adenoma vs. Advanced adenoma + Colorectal cancer | | |
|---|---|---|---|---|
| | | Cutoff | Sensitivity (%) | 95% Confidence interval (%) |
| Specificity (%) | 95 | 0.87 | 61.7 | 55.6 ~ 67.8 |
| | 90 | 0.79 | 72 | 66.4 ~ 77.7 |
| | 85 | 0.74 | 75.7 | 70.3 ~ 81.1 |
| | 80 | 0.64 | 81.9 | 77.1 ~ 86.7 |
| AUC | | 0.90 | | |

Further, when the cutoff value is set to achieve a specificity of 90%, Table 3C shows the test sensitivity and AUC values for advanced adenoma, and Table 3D shows the test sensitivity and AUC values for advanced adenoma and colorectal cancer. For the results on the left in Table 3C, the dependent variable was defined as the presence or absence of disease (control + non-advanced adenoma vs. advanced adenoma). The predicted values were then calculated by substituting each variable into the corresponding independent variable term of the model Equation (III), thereby determining the test sensitivity. For the results on the right in Table 3C, a prediction model equation was derived by setting the dependent variable as the presence or absence of disease (control + non-advanced adenoma vs. advanced adenoma), with the independent variables being the same as those used to derive Equation (IV). The predicted values were then calculated by substituting each variable into the corresponding independent variable term of the prediction model equation, thereby determining the test sensitivity. Further, for the results on the left in Table 3D, a prediction model equation was derived by defining the dependent variable as the presence or absence of disease (control + non-advanced adenoma vs. advanced adenoma + colorectal cancer), with the independent variables being the same as those used to derive Equation (III). The predicted values were then calculated by substituting each variable into the corresponding independent variable term of the prediction model equation, thereby determining the test sensitivity. Also, for the results on the right in Table 3D, the dependent variable was defined as the presence or absence of disease (control + non-advanced adenoma vs. advanced adenoma + colorectal cancer). The predicted values were then calculated by substituting each variable into the corresponding independent variable term of the model Equation (IV), thereby determining the test sensitivity.

**[Table 3C]**

| **Control + Non-advanced adenoma vs. Advanced adenoma** | | | | | | |
|---|---|---|---|---|---|---|
| | Multivariate analysis parameters | | | | | |
| | Normalized mSST value (continuous variable) + binarized fecal occult blood test result + age + gender | | | Binarized feces DNA test result (mSST value + normalized mSST value) + binarized fecal occult blood test result + age | | |
| | Cutoff | Sensitivity (%) | 95% Confidence interval (%) | Cutoff | Sensitivity (%) | 95% Confidence interval (%) |
| Specificity 90% | 0.81 | 46.2 | 37.1 ~ 55.2 | 0.79 | 56.4 | 47.4 ~ 65.4 |
| AUC | 0.84 | | | 0.85 | | |

**[Table 3D]**

| **Control + Non-advanced adenoma vs. Advanced adenoma + Colorectal cancer** | | | | | | |
|---|---|---|---|---|---|---|
| | Multivariate analysis parameters | | | | | |
| | Normalized mSST value (continuous variable) + binarized fecal occult blood test result + age + gender | | | Binarized feces DNA test result (mSST value + normalized mSST value) + binarized fecal occult blood test result + age | | |
| | Cutoff | Sensitivity (%) | 95% Confidence interval (%) | Cutoff | Sensitivity (%) | 95% Confidence interval (%) |
| Specificity 90% | 0.81 | 65.8 | 59.9 ~ 71.8 | 0.79 | 72 | 66.4 ~ 77.7 |
| AUC | 0.89 | | | 0.90 | | |

In the combined test of the univariate analysis (i)-3, when the control group + non-advanced adenoma group was used as the reference group, the specificity was 79.5%, and the sensitivity for advanced adenoma + colorectal cancer was 78.6% (Table 2). In contrast, when the above prediction model equation was used in the multivariate analysis, as described above, the sensitivity at a specificity of 80% was 81.9%, demonstrating an improvement in the test sensitivity of 3.3% without reducing specificity (Table 3B).

Further, Table 4 shows the results of comparison of sensitivity and specificity between the method of the present invention and conventional techniques including the fecal occult blood test and Cologuard. In Table 4, the sensitivity and specificity of Cologuard are based on the data described in the above Non-patent literature 2. In Table 4, "Binarized fecal occult blood test result + binarized fecal DNA test result (combined)" represents the combined test results shown in Table 2. Further, in Table 4, "Binarized fecal occult blood test result + binarized fecal DNA test result (multivariate analysis)" represents sensitivity and specificity obtained by performing ROC analysis using the predicted values calculated based on the above Equation (IV).

**[Table 4]**

| | | Sensitivity (%) - proportion of disease group that tests positive | | | Specificity (%) - proportion of non-disease group that tests | |
|---|---|---|---|---|---|---|
| | | Colorectal cancer Sensitivity = number of test-positive colorectal cancer patients/total number of colorectal cancer patients | Advanced adenoma Sensitivity = number of test-positive advanced adenoma patients/total number of advanced adenoma patients | Non-advanced adenoma Sensitivity = number of test-positive non-advanced adenoma patients/total number of non-advanced adenoma patients | Control Specificity = number of test-negative control subjects/total number of control subjects | Control + Non-advanced adenoma Specificity = number of test-negative subjects in Control group + Non-advanced adenoma group/total number of subjects in Control + Non-advanced adenoma |
| Fecal occult blood test | | 91.3 | 29.1 | 7 | 87.3 | 89.3 |
| Cologuard | | 92.3 | 42.4 | 17.2 | 89.8 | 86.6 |
| Binarized fecal occult blood test result + binarized feces DNA test result (combined) | | 94.4 | 61.5 | 20.9 | 79.7 | 79.5 |
| Binarized fecal occult blood test result + binarized feces DNA test result (multivariate analysis) | Specificity 90% | 86.5 | 56.4 | 11.6 | 92.4 | 91.0 |
| | Specificity 85% | 88.9 | 61.5 | 16.3 | 87.3 | 86.1 |

From Table 4, it is confirmed that the fecal occult blood test combined with the fecal DNA test using mSST exhibits higher test sensitivity for advanced adenoma than the conventional techniques. Efficient detection of advanced adenoma can help identify individuals who truly require detailed examinations such as colonoscopy.

## Claims

1. A method for assisting in diagnosis of advanced colorectal adenoma in a subject, comprising:
(i) a step A of measuring a methylation level of CpG present in a region from +1 to +300 relative to a transcription start site of a somatostatin (SST) gene in DNA extracted from feces collected from a subject;
(ii) a step B of measuring a quantitative level of a predetermined internal control gene in DNA extracted from feces collected from the subject; and
(iii) a step C of calculating a normalized methylation level by normalizing the methylation level of CpG in the SST gene measured in the step A by the quantitative level of the predetermined internal control gene measured in the step B, wherein the normalized methylation level calculated in the step C is compared with a predetermined cutoff value, thereby assisting in the diagnosis of advanced colorectal adenoma in the subject.

2. The method according to claim 1, wherein, in the step C, the normalized methylation level is calculated by dividing the methylation level of CpG in the SST gene measured in the step A by the quantitative level of the predetermined internal control gene measured in the step B.

3. The method according to claim 1 or 2, wherein the quantitative level of the internal control gene is a copy number of the internal control gene.

4. The method according to any of claims 1 to 3, wherein, in the step A, the methylation level of CpG present in the region from +1 to +300 relative to the transcription start site of the SST gene is measured by treating DNA with at least one restriction enzyme selected from the group consisting of HhaI, HpaII, BstUI, BmgT120I, AatII, BssHII, Cfr10I, HaeII, Hin1I, SacII, and VpaK11BI, and then amplifying a region containing CpG recognized by the restriction enzyme.

5. The method according to any of claims 1 to 4, wherein, in the step A, cytosine of CpG present in the region from +1 to +300 relative to the transcription start site of the SST gene is cytosine located at position 187,670,340 or 187,670,342 on human chromosome 3 (Chr3).

6. The method according to any of claims 1 to 5, wherein the subject is a subject determined to be negative by a fecal occult blood test.

7. A method for assisting in diagnosis of advanced colorectal adenoma or colorectal cancer, comprising:
(i) a step A of measuring a methylation level of CpG present in a region from +1 to +300 relative to a transcription start site of a somatostatin (SST) gene in DNA extracted from feces collected from a subject;
(ii) a step B of measuring a quantitative level of a predetermined internal control gene in DNA extracted from feces collected from the subject;
(iii) a step C of calculating a normalized methylation level by normalizing the methylation level of CpG in the SST gene measured in the step A by the quantitative level of the predetermined internal control gene measured in the step B; and
(iv) a step D of performing a fecal occult blood test and determining whether the test is positive or negative based on a predetermined cutoff value, wherein multivariate analysis is performed using, as indicators, the methylation level measured in the step A and/or the normalized methylation level calculated in the step C, and a result of the fecal occult blood test determined in the step D, thereby assisting in the diagnosis of advanced colorectal adenoma or colorectal cancer.

8. The method according to claim 7, wherein, in the step C, the normalized methylation level is calculated by dividing the methylation level of CpG in the SST gene measured in the step A by the quantitative level of the predetermined internal control gene measured in the step B.

9. The method according to claim 7 or 8, wherein, in the step A, the methylation level of CpG present in the region from +1 to +300 relative to the transcription start site of the SST gene is measured by treating DNA with at least one restriction enzyme selected from the group consisting of HhaI, HpaII, BstUI, BmgT120I, AatII, BssHII, Cfr10I, HaeII, Hin1I, SacII, and VpaK11BI, and then amplifying a region containing CpG recognized by the restriction enzyme.

10. The method according to any of claims 7 to 9, wherein cytosine of CpG present in the region from +1 to +300 relative to the transcription start site of the SST gene is cytosine located at position 187,670,340 or 187,670,342 on human chromosome 3 (Chr3).

11. A method for assisting in diagnosis of advanced colorectal adenoma or colorectal cancer in a subject, comprising:
(i) a step A of measuring a methylation level of CpG present in a region from +1 to +300 relative to a transcription start site of a somatostatin (SST) gene in DNA extracted from feces collected from a subject;
(ii) a step B of measuring a quantitative level of a predetermined internal control gene in DNA extracted from feces collected from the subject;
(iii) a step C of calculating a normalized methylation level by normalizing the methylation level of CpG in the SST gene measured in the step A by the quantitative level of the predetermined internal control gene measured in the step B;
(iv) a step D of performing a fecal occult blood test and determining whether the test is positive or negative based on a predetermined cutoff value;
(v) a step E of determining whether the methylation level measured in the step A is positive or negative based on a predetermined cutoff value; and
(vi) a step F of determining whether the normalized methylation level calculated in the step C is positive or negative based on a predetermined cutoff value, wherein the subject is determined, for the purpose of diagnostic assistance, as having advanced colorectal adenoma or colorectal cancer if a result determined in the step D and at least one of a result determined in the step E and a result determined in the step F, is positive.

12. The method according to claim 11, wherein, in the step C, the normalized methylation level is calculated by dividing the methylation level of CpG in the SST gene measured in the step A by the quantitative level of the predetermined internal control gene measured in the step B.
